Europäisches Patentamt

European Patent Office

Office européen des brevets

(11) Veröffentlichungsnummer: **0 504 810 A1**

# EUROPÄISCHE PATENTANMELDUNG

(21) Anmeldenummer: **92104599.3**

(22) Anmeldetag: **17.03.92**

(51) Int. Cl.5: **G01N 33/569**, G01N 33/58

(30) Priorität: **18.03.91 DE 4108748**

(43) Veröffentlichungstag der Anmeldung:
**23.09.92 Patentblatt 92/39**

(84) Benannte Vertragsstaaten:
**AT BE CH DE DK ES FR GB GR IT LI LU NL PT SE**

(71) Anmelder: **BOEHRINGER MANNHEIM GMBH**
**Sandhofer Strasse 112-132**
**W-6800 Mannheim-Waldhof(DE)**

(72) Erfinder: **Haselbeck, Anton, Dr.**
**Bärenmühlweg 50**
**W-8120 Weilheim(DE)**
Erfinder: **Hösel, Wolfgang, Dr.**
**Cäsar-von-Hofacker-Strasse 10**
**W-8132 Tutzing(DE)**
Erfinder: **Mäder, Michael, Dr.**
**Weender Strasse 93**
**W-3400 Göttingen(DE)**
Erfinder: **Meihorst, Dirk**
**Im Barm 1**
**W-3004 Isernhagen 2(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Kopernikusstrasse 9 Postfach 86 08**
**20**
**W-8000 München 86(DE)**

(54) **Diagnose von bakteriellen Infektionen durch Nachweis von Glykokonjugaten.**

(57) Die Erfindung betrifft ein Verfahren zur Diagnose des Auftretens oder/und des Verlaufs von bakteriellen Infektionen, insbesondere von Infektionen, die mit einer Entzündung verbunden sind, das dadurch gekennzeichnet ist, daß man eine Körperflüssigkeit mittels eines spezifischen Nachweisverfahrens für kohlenhydrathaltige Verbindungen auf das Vorhandensein von mindestens zwei Glykokonjugaten testet, die einen isoelektrischen Punkt im Bereich von pH 7 - 10 aufweisen, wobei ein positives Ergebnis dieses Tests auf eine bakterielle Infektion hinweist.

Die vorliegende Erfindung betrifft ein Verfahren zur Diagnose des Auftretens oder/und des Verlaufs von bakteriellen Infektionen, insbesondere von Infektionen, die mit einer Entzündung verbunden sind, durch den Nachweis von spezifischen Glykokonjugaten in einer Körperflüssigkeit.

Die Diagnostik bakterieller Infektionskrankheiten kann mikrobiologisch durch einen direkten Erregernachweis oder durch einen Nachweis der Immunantwort auf den Erreger (Antikörpernachweis) erfolgen. Bei akut behandlungsbedürftigen Infektionen sollte der Erreger in einer bakteriellen Kultur nachgewiesen werden. Diese Möglichkeit besteht jedoch nur, wenn sich die Erreger aus den Nativ-Präparaten (Blut, Sekret, Ausscheidungen oder Gewebe) anzüchten lassen oder direkt im Nativ-Präparat mikroskopisch bestimmbar sind.

Lassen sich die Erreger auf diese Weise nicht mehr nachweisen, so besteht die Möglichkeit, einen Antikörpertest durchzuführen. Gewöhnlich kommte es ca. 5 Tage nach der Infektion zu einem Anstieg des Antikörper-Titers über mehrere Wochen. Auf eine akute Infektion weist ein Anstieg des Antikörper-Titers auf das 2- bis 4-fache des Grenz-Titers hin. Die Antikörperbestimmung kann dabei durch Bakterienagglutination (Widal-Reaktion), indirekte Hämagglutination, Latexagglutination, Immunofluoreszenz-Tests und Enzymimmuno-Tests erfolgen.

Bekannte diagnostische Verfahren für bakterielle Infektionskrankheiten weisen jedoch einige Nachteile auf. So können beim Anzüchten erfahrungsgemäß bis zu einem genauen Nachweis des Erregers 3 bis 9 Tage vergehen. Ein sofortiger direkter Nachweis des Erregers im Nativ-Präparat gelingt nur selten und nur bei ganz bestimmten Infektionen. Erkrankungen mit chronischem Verlauf (z.B. chronische Endocarditiden) sind häufig weder mit bakteriologischen Kulturnachweisen noch durch die Bestimmung des Antikörper-Titers zu beurteilen. Bei solchen Infektionen bleibt nur die Bestimmung unspezifischer Laborparameter im Blut sowie der aufwendige Einsatz anderer diagnostischer Hilfsmittel z.B. Ultraschall, Röntgen, EKG, etc..

Aufgabe der vorliegenden Erfindung war es daher, ein Verfahren zur Diagnose bakterieller Infektionen bereitzustellen, bei dem die Nachteile des Standes der Technik mindestens teilweise beseitigt sind.

Die erfindungsgemäße Aufgabe wird gelöst durch ein Verfahren zur Diagnose des Auftretens oder/und des Verlaufs von bakteriellen Infektionen, insbesondere von Infektionen, die mit einer Entzündung verbunden sind, wobei man eine Körperflüssigkeit mittels eines spezifischen Nachweisverfahrens für kohlenhydrathaltige Verbindungen auf das Vorhandensein von mindestens zwei Glykokonjugaten testet, die einen isoelektrischen Punkt im Bereich von pH 7 - 10 aufweisen, wobei ein positives Ergebnis dieses Tests auf eine bakterielle Infektion hinweist.

Überraschenderweise wurde festgestellt, daß in Körperflüssigkeiten, insbesondere in Serum oder in der Cerebrospinalflüssigkeit von Patienten mit bakteriellen Infektionen, insbesondere von bakteriellen Infektionen mit entzündlichem Charakter, mindestens 2 Glykokonjugate auftreten, die einen isoelektrischen Punkt im Bereich von pH 7 - 10 aufweisen. Personen mit einem positiven Testergebnis haben mit hoher Wahrscheinlichkeit eine bakterielle Infektion. Erkrankungen, die nicht bakteriellen Ursprungs sind, wie etwa virale Infektionen, Autoimmunerkrankungen, Infarkte und Gefäßprozesse sind nicht mit dem Auftreten dieser speziellen Glykokonjugate assoziiert. Durch das erfindungsgemäße Verfahren gelingt es daher, auf einfache Weise bakterielle Erreger in Körperflüssigkeiten indirekt nachzuweisen.

Diese Glykokonjugate sind zwar nicht bei sämtlichen bakteriellen Infektionen nachzuweisen, werden aber insbesondere bei bakteriellen Infektionen mit nicht identifizierbaren Erregern, die mit entzündlichen Prozessen verbunden sind (Abszesse, Fisteln, etc.), gefunden. Weiter wurde festgestellt, daß auch bei bislang schwer zu diagnostizierenden chronischen Infektionen das erfindungsgemäße Testverfahren erfolgreich einsetzbar ist.

Die Bandenmuster der Glykokonjugate nach isoelektrischer Fokussierung sind bei verschiedenen Infektionen unterschiedlich, so daß sogar von einem bestimmten Bandenmuster auf eine bestimmte Infektionskrankheit geschlossen werden kann. Das erfindungsgemäße Verfahren eignet sich sowohl zur frühzeitigen Erkennung einer bakteriellen Infektion als auch zur Verlaufskontrolle, da es beim Abklingen der Infektion wieder verschwindet.

Das erfindungsgemäße Verfahren beinhaltet einen Test auf Glykokonjugate mit einem isoelektrischen Punkt im Bereich von pH 7 bis 10. Unter einem Glykokonjugat im Sinne der Erfindung versteht man eine kohlenhydrathaltige Verbindung auf Basis eines Proteins und/oder Lipids, aber auch ein Polysaccharid. Glykokonjugate, die eine unterschiedliche Ladung tragen, können sich demnach sowohl in ihrem Protein-, Lipid- und Kohlenhydratanteil als auch nur in Teilen davon unterscheiden.

In Körperflüssigkeiten des Menschen findet man eine große Anzahl verschieden glykosylierter Produkte. Der erste Schritt bei der Durchführung des erfindungsgemäßen Verfahrens ist es daher, diese glykosylierten Produkte in Abhängigkeit von ihrem isoelektrischen Punkt, d.h. in Abhängigkeit von ihrer elektrischen Gesamtladung, aufzutrennen. Für diesen Verfahrensschritt sind eine Vielzahl von Trennungsmethoden bekannt, die eine Auftrennung von Makromolekülen in Abhängigkeit des pH-Werts ermöglichen. Beispielsweise kann eine solche Auftrennung über selektive Ionenaustauscher erfolgen. Besonders bevorzugt für das erfindungsgemäße Verfahren ist jedoch eine Auftrennung über isoelektrische Fokussierung. Die isoelektrische Fokussierung ist ein allgemein bekanntes Verfahren und kann beispielsweise nach Schipper et al. (Science Tools 31 (1984), 5-6) durchgeführt werden. Vorzugsweise wird die isoelektrische Fokussierung in Gegenwart von hohen Harnstoffkonzentrationen (z.B. 4 mol/l) durchgeführt, um eine unspezifische Bandierung von hochglykosylierten Makromolekülen weitestgehend zu vermeiden.

Der zweite Schritt bei der Durchführung des erfindungsgemäßen Verfahrens ist ein spezifischer Nachweis für kohlenhydrathaltige Verbindungen. Dieser Schritt ist erforderlich, da bei Auftrennung nach dem isoelektrischen Punkt viele Makromoleküle im Bereich zwischen pH 7 und 10 vorliegen, die jedoch keine Reaktion mit Kohlenhydratnachweisreagenzien zeigen. Die Durchführung des Kohlenhydratnachweises erfolgt nach an sich bekannten Verfahren.

So ist es z.B. aus der DE-OS 36 29 194 bekannt, Kohlenhydrat-Reste in Glykokonjugaten durch Reaktion mit einem Biotin-Derivat unter anschließender Umsetzung mit einem markierten Streptavidin nachzuweisen.

Eine bevorzugte Methode für den Nachweis von Kohlenhydraten bei dem erfindungsgemäßen Verfahren ist in der EP-A 0 347 946 offenbart. Dort ist ein Verfahren zum Nachweis von kohlenhydrathaltigen Verbindungen beschrieben, worin man die nachzuweisende Verbindung mit einer Verbindung umsetzt, die eine Gruppierung, die eine spezifische Bindung an den Kohlenhydratteil der nachzuweisenden Substanz ermöglicht und ein Hapten mit einem Molekulargewicht von 300 bis 1200 enthält, anschließend den gebildeten Komplex mit markierten, gegen das Hapten gerichteten Antikörpern in Kontakt bringt und die Markierung in an sich bekannter Weise bestimmt.

Durch dieses Verfahren lassen sich insbesondere Pentosen, Hexosen, Sialinsäure und deren Derivate in Glykokonjugaten nachweisen.

Als Gruppierung, die spezifisch mit den Glykokonjugaten reagiert, kann in einer bevorzugten Ausführungsform des erfindungsgemäßen Verfahrens ein Lectin oder ein Gemisch von Lectinen verwendet werden (Biochem. J. 245 (1987), 1-11, Goldstein I.J. und Hayes, C.E. (1978) in Advan. Carbohydr. Chem. Biochem. (Tipson, R.S. und Horton, D. Hrsg.) 35, 127-340, Academic Press, New York-San Francisco-London). Es sind verschiedene Lectine bekannt, die mit einzelnen Zuckerarten spezifisch binden (Ann.Rev.Biochem. 55 (1986), 35-67). Diese Lectine können aus Pflanzen isoliert werden und sind im Handel (u.a. von Boehringer Mannheim GmbH, Deutschland) erhältlich. Verwendet werden können z.B. Peanut-Agglutinin (PNA), das spezifisch an endständige Galactose-Reste bindet, Wheat germ-Agglutinin (WGA), das spezifisch für N-Acetylglucosamin ist, Galanthus nivalis-Agglutinin (GNA), das spezifisch mit endständigen Mannose-Resten bindet, Sambucus nigra-Agglutinin (SNA), das spezifisch für $\alpha$(2-6) gebundene Sialinsäuren ist. Besonders bevorzugt ist das Maackia amurensis-Lectin (MAA)(R.N. Knibbs et al., J.Biol.Chem. 266 (1991) 83-88), das spezifisch für $\alpha$(2-3)gebundene Sialinsäure-Reste ist. Vorzugsweise verwendet man für das erfindungsgemäße Verfahren mehrere dieser oder anderer geeigneter Lectine.

Eine weitere Möglichkeit für den spezifischen Nachweis von Kohlenhydraten besteht in einer Oxidation der nachzuweisenden Verbindung und der Verwendung einer Gruppierung, die mit dem entstehenden, spezifisch oxidierten Rest, in der Regel einer Aldehyd– oder Carboxylgruppe, reagiert. Bei einem derartigen Nachweis wird vorzugsweise der Kohlenhydratteil der zu bestimmenden Verbindung mit einem spezifischen Enzym (z.B. Glucoseoxidase oder Galactoseoxidase) oder mit Perjodat oxidiert. Dabei entstehen Aldehydgruppen. Als damit bindefähige funktionelle Gruppierungen sind Amine und Hydrazine geeignet. Im Falle der Verwendung eines Amins als funktionelle Gruppierung werden die dabei entstehenden Schiff'schen Basen besonders bevorzugt anschließend an diese Umsetzung noch dehydriert, um die instabile Aldimin-Bindung in eine stabile Aminbindung zu überführen.

Derartige Verfahren sind in der EP-A 0 347 946 beschrieben, auf deren Offenbarung hier Bezug genommen wird.

Weiterhin ist es bevorzugt, wenn das Nachweisreagenz als Hapten Digoxin oder Digoxigenin enthält. Die Markierung des gegen das Hapten gerichteten Antikörpers kann eine radioaktive Markierung, eine Markierung mit einem Enzym, einer fluoreszierenden, chemi- oder biolumineszierenden Substanz, einer NMR-aktiven Substanz oder eine Markierung mit Gold sein. Verwendet man als Markierung ein Enzym, so ist Peroxidase oder alkalische Phosphatase bevorzugt.

In einer bevorzugten Ausführungsform der vorliegenden Erfindung werden die Gesamtserum-Proteine durch isoelektrische Fokussierung in einem pH-Bereich von 6,5 bis 9,5 aufgetrennt, auf eine geeignete Membran (Nitrocellulose, Polyvinylidendifluorid, Nylon) transferiert und die kohlenhydrathaltigen Verbindungen mit Hilfe des oben genannten immunologischen Verfahrens (EP-A 0 347 946) nachgewiesen. Als besonders geeignet haben sich der allgemeine Kohlenhydrat-Nachweis mit Hilfe von Digoxigenin-succinyl-$\epsilon$-aminocapronsäurehydrazid nach chemischer Oxidation der Zuckerreste mit Metaperjodat, eine enzymatische Oxidation der N-ständigen Galactosereste mit Galactose-Oxidase sowie die Reaktion mit dem digoxigenylierten Lectin aus Maackia amurensis (MAA) erwiesen.

Die Erfindung soll durch die folgenden Beispiele in Verbindung mit der Zeichnung verdeutlicht werden.

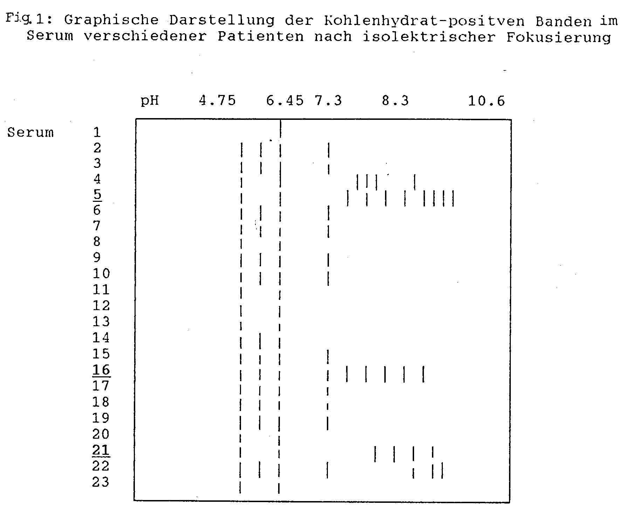

Fig. 1 stellt die graphische Darstellung eines Blots der Sera von 23 Personen nach isoelektrischer Fokussierung und spezifischem Glykannachweis dar.

Beispiel 1

Allgemeiner Kohlenhydrat-Nachweis

Serumproben werden durch isolelektrische Fokussierung in einem Gelsystem, (vgl. Schipper et.al.), aufgetrennt und durch Druck-Diffusions Transfer (nach Bowen et al. Nucl. Acids Res. 1980, 8, 1-3) auf Nitrozellulose (BA 85, Schleicher & Schüll) gebracht. Die so erhaltene Nitrocellulose-Membran wird nach Waschen in PBS (50 mmol/l Kaliumphosphat Puffer, pH 6.5, 150 mmol/l Natriumchlorid) für 20 Minuten bei Raumtemperatur mit 10 mmol/l Natrium-Metaperjodat in 0.1 mol/l Natrium-Acetat Puffer, pH 5.5 inkubiert, dreimal je 5 Minuten mit PBS gewaschen und dann 60 Minuten bei Raumtemperatur mit 1 $\mu$mol/l Digoxigenin-succinyl-$\epsilon$-aminocapronsäu-rehydrazid in 0,1 mol/l Natrium-Acetat Puffer, pH 5.5 inkubiert.

Nach Waschen mit TBS (50 mmol/l Tris-HCl, pH 7.5, 150 mmol/l Natriumchlorid) wird die Membran für 30 Minuten mit 0.5% Casein in TBS behandelt und anschließend mit Anti-Digoxigenin Antikörper, konjugiert mit alkalischer Phosphatase (Aktivität der alkalischen Phosphatase: 0,75 U/ml) in TBS für 60 Minuten inkubiert. Nach erneutem Waschen mit TBS wird die Nachweisreaktion auf alkalische Phosphatase mit dem Substrat 5-Brom-4-Chlor-3-Indolylphosphat und Nitroblau Tetrazoliumchlorid durchgeführt. Kohlenhydrat-enthaltende Verbindungen erscheinen als grau-schwarze Banden. Zum Stoppen der Reaktion wird die Membran mit Wasser gespült und getrocknet.

Beispiel 2

Nachweis mit dem Lectin aus Maackia amurensis

Die Auftrennung der Serumproteine und der Transfer auf Nitrozellulose erfolgt, wie in Beispiel 1 beschrieben.

Die Membran wird mit 0.5 % Casein in TBS für 30 Minuten behandelt, zweimal mit TBS und einmal mit TBS, das 1 mmol/l $CaCl_2$, 1 mmol/l $MgCl_2$, 1 mmol/l $MnCl_2$ enthält, gewaschen.

Sie wird anschließend mit 1 $\mu$g/ml Maackia amurensis Lectin (MAA), an welches Digoxigenin gebunden ist, in TBS für 60 Minuten bei Raumtemperatur inkubiert und dann wiederum mit TBS gewaschen.

Die Detektion des Digoxigenins erfolgt wie in Beispiel 1 beschrieben.

Diese Art von Analytik ist prinzipiell nicht auf Membranen als Träger beschränkt, sondern läßt sich auch in Mikrotiterplatten oder anderen geeigneten Trägern (Röhrchen etc.) durchführen.

Beispiel 3

Glykokonjugatmuster im Serum und in der Cerebrospinalflüssigkeit

Es wurden die Seren von 23 Personen auf die Anwesenheit von Glykokonjugaten getestet. Der Glykokonjugat-Nachweis erfolgte gemäß Beispiel 2, außer daß 6 Lectine mit unterschiedlicher Spezifität, konjugiert an Digoxigenin, verwendet wurden. Die Lectine waren AAA (Aleuria aurantia-Agglutinin, spezifisch für Fucose), DSA (Datura stramonium Agglutinin, spezifisch für Galactose $\beta$ (1-4) N-acetylglucosamin), GNA (Galanthus nivalis-Agglutinin, spezifisch für terminale Mannose $\alpha$(1-3), $\alpha$(1-6), $\alpha$(1-2)), MAA (Maackia amurensis Lectin, spezifisch für Sialinsäure $\alpha$(3-2) Galactose, PNA (Peanut-Agglutinin, spezifisch für Galactose $\beta$(1-3) N-Acetylgalactosamin) und SNA (Sambucus nigra-Agglutinin, spezifisch für Sialinsäure $\alpha$-(2-6) galactose)(Hersteller: Boehringer Mannheim GmbH).

In der Zeichnung ist der Blot dieser Seren dargestellt, wobei die Seren gemäß Beispiel 1 zuerst durch isoelektrische Fokussierung aufgetrennt und nach dem Blotten durch den Glykannachweistest untersucht wurden. 3 Seren (Nr. 5, 16 und 21) zeigen das typische Glykanmuster, das mit massiven bakteriellen Infektionen assoziiert ist. 3 Seren (Nr. 4, 22 und 23) zeigen leicht entwickelte Muster, die leichteren bakteriellen Infektionen zugeordnet werden konnten.

Bei Untersuchungen mit einer größeren Anzahl von Seren wurde das Glykanmuster in 7 % der Seren gefunden, sowohl bei Patienten mit neurologischen (N = 300) als auch inneren (N = 100) Störungen.

Proben des Serums und der Cerebrospinalflüssigkeit zeigten jeweils identische Muster. 100 Seren einer Referenzgruppe (Proben aus einer Blutbank) zeigten kein Glykanmuster.

Beispiel 4

Zuordnung von Patientenseren zu bestimmten Krankheitsgruppen

Untersuchungen mit Seren einer definierten Patientengruppe haben ergeben, daß die Glykokonjugatmuster insbesondere bei bakteriellen Infektionen auftreten, nicht jedoch bei viralen Infektionen, Autoimmunerkrankungen, Infarkten und Gefäßprozessen. Die nachfolgenden Tabellen 1 und 2 zeigen die Zuordnung der analog Beispiel 1 und 2 untersuchten Patientenseren zu bestimmten Krankheitsgruppen:

Tabelle 1

| Krankheitsgruppen | Anzahl der Fälle | Anzahl der Fälle mit typischen Bandenmustern im Glykokonjugat-Nachweis: |
|---|---|---|
| nicht entzündliche Herz-Kreislauf-Gefäßerkrankungen | 40 | 0 |
| Autoimmunerkrankungen (Rheumat. Formenkreis, Lupus erythematodes, Kollagenosen) | 33 | 1 |
| Andere chron. entzündl. nicht bakt. Erkrankungen (Encephalitis diss.) | 5 | 0 |
| Tumorerkrankungen | 6 | 0 |
| Virale Infektionen | 30 | 1 |
| Bakt. Infektionen | 15 | 9 |
| Andere nicht infektiöse Erkrankungen (Migräne, Lumbago, Epilepsie) | 10 | 0 |
| Summe | 139 | 11 |

Tabelle 2 zeigt eine Aufschlüsselung der bakteriellen Infektionen nach akuten und chronischen Erkrankungen und die damit verbundene Häufigkeit des Auftretens des Bandenmusters im Glykokonjugatnachweis.

Tabelle 2

| | Anzahl d. Fälle | Mit Bandenmuster | Häufigkeit in % |
|---|---|---|---|
| Akute Infektion | 11 | 6 | 55 |
| Chron. Infektion | 4 | 3 | 75 |

Bislang wurden bei Infektionen mit Pseudomonas aeruginosa, Serratia, Streptococcus viridans, Neisseria meningitidiae, Listerien sowie insbesondere bei bakteriellen Infektionen mit nicht identifizierbaren Erregern, die zur Bildung von Abszessen oder Fisteln führten, Banden im Glykokonjugat-Test gefunden.

**Patentansprüche**

1. Verfahren zur Diagnose des Auftretens oder/und des Verlaufs von bakteriellen Infektionen, insbesondere von Infektionen, die mit einer Entzündung verbunden sind,
   **dadurch gekennzeichnet,**
   daß man eine Körperflüssigkeit mittels eines spezifischen Nachweisverfahrens für kohlenhydrathaltige Verbindungen auf das Vorhandensein von mindestens zwei Glykokonjugaten testet, die einen isoelektrischen Punkt im Bereich von pH 7 - 10 aufweisen, wobei ein positives Ergebnis dieses Tests auf eine bakterielle Infektion hinweist.

2. Verfahren nach Anspruch 1,
   **dadurch gekennzeichnet,**
   daß vor dem spezifischen Nachweis für kohlenhydrathaltige Verbindungen die gesuchten Glykokonjugate von Glykokonjugaten mit anderen isoelektrischen Punkten abgetrennt werden.

3. Verfahren nach einem der vorhergehenden Ansprüche,
   **dadurch gekennzeichnet,**
   daß die Glykokonjugate immobilisiert werden, bevor der spezifische Nachweis für kohlenhydrathaltige Verbindungen durchgeführt wird.

4. Verfahren nach einem der vorhergehenden Ansprüche,
   **dadurch gekennzeichnet,**
   daß man zum spezifischen Nachweis für kohlenhydrathaltige Verbindungen die Glykokonjugate mit einem Nachweisreagenz umsetzt, das eine Gruppierung, die eine spezifische Bindung an den Kohlenhydrat-Teil von Glykokonjugaten ermöglicht, und ein Hapten mit einem Molekulargewicht von 300 bis 1200 enthält, anschließend den gebildeten Komplex mit markierten, gegen das Hapten gerichteten Antikörpern in Kontakt bringt und die Markierung bestimmt.

5. Verfahren nach Anspruch 4,
   **dadurch gekennzeichnet,**
   daß man als Gruppierung, die eine spezifische Bindung an die nachzuweisende Substanz ermöglicht, ein Lectin oder ein Gemisch von unterschiedlichen Lectinen verwendet.

6. Verfahren nach Anspruch 5,
   **dadurch gekennzeichnet,**
   daß man als Gruppierung das Lectin aus Maackia amurensis verwendet.

7. Verfahren nach Anspruch 4,
   **dadurch gekennzeichnet,**
   daß man die nachzuweisende Substanz vor Umsetzung mit dem Nachweisreagenz oxidiert.

8. Verfahren nach Anspruch 7,
   **dadurch gekennzeichnet,**
   daß man als Gruppierung, die eine spezifische Bindung an die nachzuweisende Substanz ermöglicht, eine Amin- oder eine Hydrazidgruppe verwendet.

9. Verfahren nach einem der Ansprüche 4 bis 8,
   **dadurch gekennzeichnet,**
   daß man als Hapten Digoxin oder Digoxigenin verwendet.

10. Verfahren nach einem der Ansprüche 4 bis 9,
    **dadurch gekennzeichnet,**
    daß man als Markierung des gegen das Hapten gerichteten Antikörpers eine radioaktive Markierung, ein Enzym, eine fluoreszierende, chemi- oder biolumineszierende Substanz, eine NMR-aktive Substanz oder Gold verwendet.

6

Fig. 1: Graphische Darstellung der Kohlenhydrat-positven Banden im
Serum verschiedener Patienten nach isolektrischer Fokusierung

Europäisches Patentamt

**EUROPÄISCHER RECHERCHENBERICHT**

Nummer der Anmeldung

EP    92 10 4599
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 374 946 (BOEHRINGER MANNHEIM GMBH) <br> * das ganze Dokument * <br> --- | 1-10 | G01N33/569 <br> G01N33/58 |
| D,A | DE-A-3 629 194 (YEDA RESEARCH AND DEVELOPMENT CO. LTD.) <br> * Seite 3, Zeile 65 - Seite 4, Zeile 51 * <br> * Seite 12, Zeile 60 - Seite 13, Zeile 2; Ansprüche 1-9,11,12 * <br> --- | 1-10 | |
| A | DE-A-3 807 440 (PROGEN BIOTECHNIK GMBH) <br> * Seite 1, Zeile 22 - Zeile 34 * <br> * Seite 2, Zeile 62 - Zeile 67 * <br> * Seite 4, Zeile 29 - Zeile 50 * <br> * Seite 5, Zeile 57 - Zeile 64 * <br> * Ansprüche 1-7,9-11,23 * <br> --- | 1-10 | |
| D,A | THE JOURNAL OF BIOLOGICAL CHEMISTRY <br> Bd. 266, Nr. 1, 5. Januar 1991, BALTIMORE, MD US <br> Seiten 83 - 88; <br> R.N. KNIBBS ET AL.: 'Characterisation of the Carbohydrate Binding Specifity of the Leukoagglutinating Lectin from Maackia amurensis' <br> * Zusammenfassung * <br> * Seite 87, linke Spalte, Zeile 21 - Seite 88, linke Spalte, Zeile 21 * <br> --- | 1,2,5,6 | |
| D,A | ANNUAL REVIEWS IN BIOCHEMISTRY <br> Bd. 55, 1986, PALO ALTO, CA, US <br> Seiten 35 - 67; <br> HALINA LIS AND NATHAN SHARON: 'Lectins as Molecules and as Tools' <br> * Seite 56, Zeile 34 - Seite 58, Zeile 16 * <br> * Seite 59, Zeile 40 - Seite 60, Zeile 6 * <br> --- | 1-10 | **RECHERCHIERTE SACHGEBIETE (Int. Cl.5)** <br><br> G01N |
| A | EP-A-0 209 155 (BOEHRINGER MANNHEIM GMBH) <br><br> * Seite 2, Spalte 1, Zeile 1 - Seite 3, Spalte 4, Zeile 26; Ansprüche 1-5 * | 1,4,7, 8-10 | |

Der vorliegende Recherchenbericht wurde für alle Patentansprüche erstellt

| Recherchenort | Abschlußdatum der Recherche | Prüfer |
|---|---|---|
| DEN HAAG | 11 JUNI 1992 | DöPFER, K.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................................
& : Mitglied der gleichen Patentfamilie, übereinstimmendes Dokument

EPO FORM 1503 03.82 (P0403)

Europäisches
Patentamt

**EUROPÄISCHER RECHERCHENBERICHT**

Nummer der Anmeldung

EP   92 10 4599
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| | ----- | | |
| | | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| DEN HAAG | 11 JUNI 1992 | DöPFER, K.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..............................................................
& : Mitglied der gleichen Patentfamilie, übereinstimmendes Dokument

EPO FORM 1503 03.82 (P0403)